# EUROPEAN PATENT SPECIFICATION

(11) **EP 4 262 943 B1**
(45) Date of publication and mention of the grant of the patent: **07.08.2024**
(21) Application number: 21839916.0
(22) Date of filing: 15.12.2021
(51) Int. Cl.: A61M 15/00

(54) **DETECTING THE PRESENCE OF LIQUID IN A VIBRATING MEMBRANE NEBULIZER**
NACHWEIS DER ANWESENHEIT VON FLÜSSIGKEIT IN EINEM SCHWINGMEMBRANVERNEBLER
DÉTECTION DE LA PRÉSENCE DE LIQUIDE DANS UN NÉBULISEUR À MEMBRANE VIBRANTE

(30) Priority: 16.12.2020 EP 20214452
(43) Date of publication of application: 25.10.2023
(73) Proprietor: Vectura Delivery Devices Limited, Chippenham, Wiltshire SN14 6FH (GB)
(72) Inventor: WEISER, Yannic, Cambridge CB4 0GZ (GB)
(74) Representative: Clarke, Christopher John
(86) International application number: PCT/EP2021/085980
(87) International publication number: WO 2022/129220

(56) References cited:
- EP-A1- 2 047 914
- EP-A1- 2 883 564
- WO-A1-2015/091356
- DE-A1- 10 022 795
- DE-A1- 10 203 079
- US-A1- 2006 102 172

## Description

### Technical Field of the Invention

The present invention relates to a vibrating membrane nebulizer, and in particular to a method for detecting the presence of liquid in contact with the membrane.

### Background to the Invention

Aerosols for medical inhalation therapy generally comprise an active ingredient dissolved or suspended in an aerosolisable liquid, often water. A homogeneous distribution of aerosol droplets with a droplet size of around 5 µm is required in order to reach deep into the lungs. Vibrating membrane nebulizers are one type of device for producing such aerosols. These devices have an aerosol generator which comprises a vibrator, such as piezoelectric element which is excited at ultrasonic frequencies in order to induce vibration; a membrane (sometimes called a mesh or aperture plate), which has a large number of holes which typically have a diameter of 1 µm to 10 µm; and a reservoir, which supplies the liquid drug formulation to the membrane. DE10022795 and DE10203079 disclose breath-controlled vibrating membrane nebulizers.

Continuing to operate a vibrating membrane nebulizer after the liquid reservoir has been emptied can cause the membrane to crack or break. Therefore, it is important to be able to reliably detect whether or not liquid is present in the reservoir/ in contact with the membrane ("empty detection"). When the liquid has been used up, the nebulizer can indicate the end of the treatment session to the patient and / or turn off the vibrator automatically.

One approach is simply to measure the amount of liquid in the reservoir with a suitable sensor. For example, US2006/0255174 discloses a nebulizer in which the amount of liquid in the reservoir is sensed by a piezoelectric sensor, an optical sensor, a conductivity sensor or a strain gauge. However this requires a dedicated sensor, which adds to the cost and complexity of the device; also, the sensor may need to be in contact with the liquid, which can present problems.

An alternative approach exploits the fact that the vibration characteristics of the aerosol generator (e.g. resonant frequency, power consumption etc.) are typically quite different when the membrane is in contact with liquid compared to when it is dry. For example, US2006/0102172 discloses a nebulizer which determines whether liquid is present or not by comparing the detected value of an electrical parameter (such as the current to the piezoelectric element) at a particular frequency with a stored value. US9272101 uses impedance measurements in a similar manner. Instead of using a single frequency, the electromechanical behaviour of the aerosol generator can be expressed as a spectrum, for example a graph of power or current consumption as a function of the vibration frequency, for example as disclosed in EP2047914. WO2014/062175 and WO2015/091356 disclose methods for comparing measured and stored spectra, using averages and various mathematical techniques, to improve the reliability of empty detection. A comparison is typically performed at regular intervals while the membrane is being vibrated during the treatment session, until it is identified that the liquid has all been used up.

However, inherent variations in the hardware components of the nebulizer (e.g. aerosol generator, driver electronics) and / or changes in these over the lifetime of the nebulizer, or due to changes in the external conditions, such as the ambient temperature, can affect the electro-mechanical behaviour. For example, methods which rely on comparisons with pre-set values can produce erroneous outcomes if the properties of the aerosol generator and / or driver electronics are differ from those onwhich the standard, stored values are based. Consequently, the vibrator could continue vibrating after the liquid has been used up, which could damage the membrane; or vibration could be stopped while liquid still remains to be aerosolized, so that the full dose is not delivered to the patient. Thus there remains a need for improved methods for reliably detecting the presence of liquid in the reservoir / or in contact with the membrane.

### Brief Description of the Invention

The present invention provides an improved method for determining when the liquid to be nebulized has been used up. The inventor recognized that transient effects can be observed in the spectra immediately after vibration starts or stops, and that these can be used to reliably detect the presence or absence of liquid in contact with the membrane. Accordingly, in a first aspect, the present invention provides a breath-actuated inhalation device comprising: an aerosol generator comprising a vibrator and a membrane; a reservoir for liquid to be aerosolized which is in fluid communication with the membrane; and a controller which provides a driver signal to drive the vibrator so that the membrane vibrates and generates an aerosol; wherein the controller is configured to:
- drive the vibrator intermittently so that the aerosol generator repeatedly has periods of aerosol generation during a patient's inhalations and periods of little or no aerosol generation preceding and/or succeeding the inhalations;
- perform scans in which the membrane is vibrated at a plurality of frequencies, and in which at least one electrical parameter of the vibrator is measured at the plurality of frequencies to provide a spectrum; wherein scans are performed during the inhalations and during the periods preceding or succeeding the inhalations;
- compare a spectrum obtained during an inhalation with a spectrum obtained during the period preceding or succeeding that inhalation;
- determine whether liquid is present in the reservoir on the basis of the comparison of the spectra; and
- cease to drive the vibrator if the controller determines that no liquid is present.

Previous empty detection methods that measure the values of an electrical parameter while the membrane is being vibrated rely on changes in the electro-mechanical properties of the aerosol generator as the volume of liquid decreases over time. In contrast, the present invention relies on an effect which occurs when the membrane is vibrated intermittently, as in a breath-actuated nebulizer. The invention compares spectra obtained during periods of aerosol generation and periods of little or no aerosol generation to identify changes which arise from the formation or dissipation of a standing wave in the reservoir when vibration is started or stopped respectively. The invention is much less dependent on the properties of aerosol generator than the previous methods because it does not rely on a comparison with, for example, a stored spectrum; instead it detects a transient change that occurs only when a liquid is present. Consequently, the invention is more robust to variations in the hardware, changes in the hardware over the lifetime of the nebulizer and changes in external conditions.

The controller may be configured to perform a first scan before each inhalation or period of aerosol generation to obtain a first spectrum; to subsequently perform a second scan during each inhalation or period of aerosol generation to obtain a second spectrum; and to compare the first and second spectra. The first scan may be performed immediately before the start of the period of aerosol generation. The second scan may be performed at least 50 or 100 ms after the start of the period of aerosol generation. This provides sufficient time for the standing wave to form, so that it is detectable in the second scan. The second scan may be performed less than 1000 ms or 500 ms after the start of the period of aerosol generation. The time delay between the first and second scans determines the point at which the absence of liquid can be detected. A smaller delay results in earlier empty detection, so the vibration of the membrane can be stopped sooner.

The inhalation device is breath-actuated, i.e. the aerosol is not generated continuously, but only when the patient inhales. The device may comprise a channel having an air inlet opening and an aerosol outlet opening, and a pressure sensor which is pneumatically connected to the channel, and the controller may be configured to: receive a signal from the pressure sensor; sense an inhalation by a patient at the aerosol outlet opening on the basis of the signal from the pressure sensor; and initiate a period of aerosol generation in response to the inhalation. The controller may be configured to initiate a period of little or no aerosol generation at a pre-set time after the period of aerosol generation was initiated. Alternatively, the controller may be configured to sense when the patient ceases to inhale on the basis of the signal from the pressure sensor and to initiate a period of little or no aerosol generation in response to the cessation of inhalation. The method of the invention is particularly suitable for breath-actuated nebulizers, because there is no need to change to the manner in which the nebulizer operates since the aerosol is inherently generated intermittently.

The aerosol generator may comprise a support member on which the vibrator and / or the membrane are mounted. The vibrator may be an annular piezoelectric element. The support element may be a transducer in the form of a hollow tubular body having a flange at or close to a first end onto which the piezoelectric element is attached, and a second end into or onto which the membrane is mounted. The device may comprise a filling chamber located above, and in fluid communication with, the support member, so that the filling chamber and the hollow tubular body together form the reservoir. Alternatively, the support member may comprise an essentially planar annulus or disk, and the membrane and / or the vibrator may be mounted on the support member, for example on opposite sides.

The controller may be configured to determine a resonant frequency of the aerosol generator from the spectra, and to drive the vibrator at the resonant frequency, or at a frequency that is related to the resonant frequency, such as a fixed offset from the resonant frequency, during the periods of aerosol generation, other than the scans.

The plurality of frequencies may comprise from about 10 or 15 kHz below the resonant frequency to about 10 or 15 kHz above the resonant frequency, for example from about 75 kHz to about 100 kHz.

The controller may be configured to perform the comparison of the spectra by calculating an overlap function. The controller may further be configured to determine that no liquid is present in the reservoir if the value of the overlap function is above a threshold value. The controller may also be configured to cease driving the vibrator if the overlap function is above the threshold for a plurality of consecutive inhalations or periods of aerosol generation, such as three or five inhalations or periods of aerosol generation. The overlap function provides a robust comparison method.

There is also disclosed (but not claimed) a method of operating an inhalation device of the invention, and/or of operating a breath-actuated inhalation device comprising an aerosol generator comprising a vibrator and a membrane, and a reservoir for liquid to be aerosolized which is in fluid communication with the membrane, the methods comprising:
a) driving the vibrator intermittently so that the aerosol generator repeatedly has periods of aerosol generation during a patient's inhalations and periods of little or no aerosol generation preceding and/or succeeding the inhalations;
b) performing scans in which the membrane is vibrated at a plurality of frequencies, and in which at least one electrical parameter of the vibrator is measured at the plurality of frequencies to provide a spectrum; wherein scans are performed during the inhalations and periods of little or no aerosol generation;
c) comparing a spectrum obtained during an inhalation with a spectrum obtained during the period preceding or succeeding that inhalation;
d) determining whether liquid is present in the reservoir on the basis of the comparison of the spectra; and
e) ceasing to drive the vibrator if it is determined in step d) that no liquid is present.

A first scan may be performed before each inhalationto obtain a first spectrum, a second scan may subsequently be performed during each inhalation to obtain a second spectrum, and the first and second spectra may be compared.

When the inhalation device comprises a channel having an air inlet opening and an aerosol outlet opening, and a pressure sensor which is pneumatically connected to the channel, the periods of aerosol generation may be initiated in response to inhalation by the patient on the basis of a signal from the pressure sensor. The periods of little or no aerosol generation may be initiated at a pre-set time after the period of aerosol generation was initiated. Alternatively, the periods of little or no aerosol generation may be initiated in response to the cessation of inhalation by the patient.

The spectra may be compared by calculating an overlap function. It may be determined that no liquid is present in the reservoir if the overlap function is above a threshold value. Driving the vibrator may cease if the overlap function is above the threshold value for a plurality of consecutive inhalations or periods of aerosol generation, such as three or five inhalations or periods of aerosol generation.

In a specific embodiment, the invention provides a breath-actuated inhalation device comprising: an aerosol generator comprising a vibrator and a membrane; a reservoir for liquid to be aerosolized which is in fluid communication with the membrane; and a controller which provides a driver signal to drive the vibrator so that the membrane vibrates and generates an aerosol in the channel; wherein the controller is configured to:
- drive the vibrator intermittently so that the aerosol generator repeatedly has periods of aerosol generation during a patient's inhalations and periods of little or no aerosol generation preceding and/or succeeding the inhalations;
- immediately before, or at the start of each inhalation or period of aerosol generation, perform a first scan in which the membrane is vibrated at a plurality of frequencies, and in which at least one electrical parameter of the vibrator is measured for each of the plurality of frequencies to provide a first spectrum;
- subsequently during each inhalation or period of aerosol generation, perform a second scan in which the membrane is vibrated at the plurality of frequencies, and in which the at least one electrical parameter of the vibrator is measured for each of the plurality of frequencies to provide a second spectrum;
- perform a comparison of the first and second spectra;
- determine whether liquid is present in the reservoir on the basis of the comparison of the first and second spectra; and
- cease to drive the vibrator if the controller determines that no liquid is present.

A method of operating a breath-actuated inhalation device according to this specific embodiment (the method not separately claimed) comprises:
a) driving the vibrator intermittently so that the aerosol generator repeatedly has periods of aerosol generation during a patient's inhalations and periods of little or no aerosol generation preceding and/or succeeding the inhalations;
b) immediately before, or at the start of each inhalation or period of aerosol generation, performing a first scan in which the membrane is vibrated at a plurality of frequencies, and in which at least one electrical parameter of the vibrator is measured for each of the plurality of frequencies to provide a first spectrum;
c) subsequently during each inhalation or period of aerosol generation, performing a second scan in which the membrane is vibrated at the plurality of frequencies, and in which the at least one electrical parameter of the vibrator is measured for each of the plurality of frequencies to provide a second spectrum;
d) performing a comparison of the first and second spectra;
e) determining whether liquid is present in the reservoir on the basis of the comparison of the first and second spectra; and
f) ceasing to drive the vibrator if it is determined in step e) that no liquid is present.

### Brief Description of the Figures

The invention will now be further described with reference to the Figures, wherein:
Figure 1 shows an expanded view of a vibrating membrane nebulizer.
Figure 2 is a cross-sectional view through the aerosol generator for the nebulizer of Figure 1.
Figure 3 is a schematic diagram of the driver circuit for the aerosol generator.
Figure 4 shows schematic drawings of the liquid surface in the reservoir before (Figure 4A) and during (Figure 4B) vibration of the membrane.
Figure 5 shows spectra obtained without liquid in the reservoir.
Figure 6 shows spectra obtained with liquid in the reservoir.
Figure 7 shows a graph of the overlap function over the course of a representative treatment.

### Detailed Description of the Invention

The term "period of aerosol generation" refers to a period of time in which the vibrator is mainly driven at the normal, intended frequency for generating an aerosol, which is typically at or near (e.g. within 2 kHz of) the resonant frequency. A period of aerosol generation may also include short periods of time in which one or more scans are performed. A period of aerosol generation may correspond to the typical length of a patient's inhalation, such as from 1 to 10s, 2 to 6s, or 3 to 5s. The term "period of little or no aerosol generation" refers to the intervals between periods of aerosol generation in which the vibrator is mainly not driven. A period of little or no aerosol generation may also include short periods of time in which one or more scans are performed. Since most of the scan frequencies are quite far (e.g. more than 2 kHz) from the resonant frequency, little or no aerosol is generated during a scan. Consequently, little or no aerosol is generated in a period of little or no aerosol generation. A period of little or no aerosol generation may correspond to the typical time between a patient's inhalations, such as from 1 to 10s, 2 to 6s, or 3 to 5s. Thus, intermittently driving the vibrator (at the normal driving frequency) results in alternate periods of aerosol generation and no aerosol generation.

The term "scan" refers to the process of sequentially vibrating the vibrator at a large number of different frequencies in stepwise increments across a defined range, and measuring the value of an electrical parameter at some or all of the frequencies. The term "spectrum" refers to a graph which is obtained by plotting the measured values of the electrical parameter as a function of frequency. The electrical parameter may be the current, voltage, power, impedance and/or the current / voltage phase shift. In particular, the electrical parameter may be the current consumption of the vibrator, or of a power converter which provides the power to the vibrator, or the voltage drop at the vibrator. These parameters can be measured by using one or more current and/or voltage sensors, in a direct or an indirect manner.

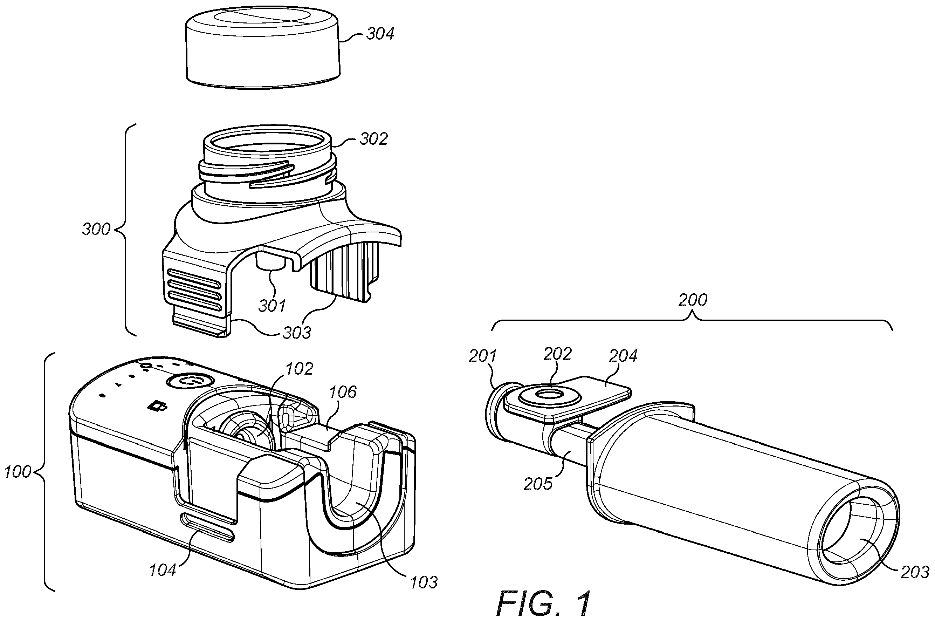

Figure 1 shows an expanded view of a vibrating membrane nebulizer device, which is described in EP2724741 and WO2013/098334. The device comprises three parts: a base unit, a mouthpiece component, and an aerosol head. The base unit **100** has one or more air inlet opening(s) in its rear end (not visible in Figure 1), an air outlet opening **102,** a groove **103** for receiving the mouthpiece component **200,** and one or more key lock members **104.** A channel within the base unit (not visible in Figure 1) connects the air inlet opening(s) to the air outlet opening **102.** The mouthpiece component **200** has an air inlet opening **201** which is attachable to the air outlet opening **102** of the base unit **100,** a lateral opening **202** for receiving an aerosol generator **301,** and an aerosol outlet opening **203.** A channel **205** extends from the air inlet opening **201** to the aerosol outlet opening **203.** The mouthpiece **200** is insertable into the groove **103** of the base unit **100.** The aerosol head **300** comprises the aerosol generator **301,** a filling chamber **302** for the liquid drug formulation to be aerosolized, which is in fluid contact with the upper end of the aerosol generator **301,** and one or more key lock members **303** complementary to the key lock members **104** of the base unit **100.** A lid **304** closes the filling chamber **302** and prevents contamination or spillage of the liquid during use.

The base unit **100,** the mouthpiece **200** and the aerosol head **300** are detachably connectible with one another. The device is assembled by inserting the mouthpiece **200** into the groove **103** in the base unit **100,** then placing the aerosol head **300** over the mouthpiece **200** and engaging the key lock member(s) **303** of the aerosol head **300** with the key lock member(s) **104** of the base unit **100** by gentle pressure on both the aerosol head and the base unit. The aerosol generator **301** is positioned in the aerosol head **300** in such a way that when engaging the key lock member(s), the aerosol generator **301** is inserted into the lateral opening **202** of the mouthpiece **200.** This creates airtight connections between the aerosol generator **301** and the lateral opening **202** in the mouthpiece as well as between the air outlet opening **102** of the base unit **100** and the air inlet opening **201** of the mouthpiece **200.** The base unit **100,** the mouthpiece **200** and the aerosol head **300** can be separated by reversing these steps.

The base unit **100** has one or more indentation(s) **106** positioned at or near the groove **103,** and the mouthpiece **200** has one or more positioning member(s) **204.** The indentation(s) of the base unit are complementary to (i.e. shaped to receive) the positioning member(s) of the mouthpiece. In this context, an indentation is a depression whose "negative" shape is complementary to the "positive" shape of a positioning member, such as a flange, projection or the like. Together, the indentations and positioning members act to position the mouthpiece correctly in the base unit. The indentation(s) and the positioning member(s) may be asymmetrical, so that the mouthpiece can only be inserted into the base unit in one way. This ensures that the device is assembled in such a manner that the position and orientation of the mouthpiece and base unit relative to each other are correct. The base unit contains a controller, such as a printed circuit board (PCB) which controls the operation of the nebulizer.

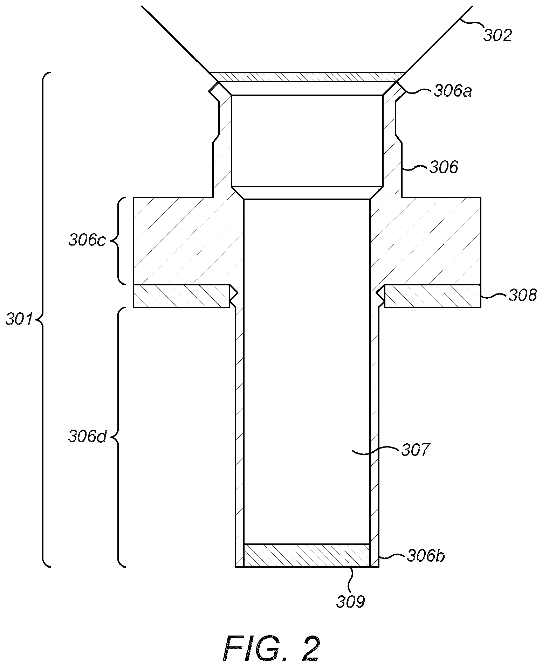

Figure 2 shows the aerosol generator, which is described in detail in WO2008/058941. It comprises a vibrator, e.g. a piezoelectric element **308,** a transducer body **306** and a membrane **309.** The piezoelectric element is preferably an annular single or multi-layer ceramic, which vibrates the transducer body in a longitudinal mode. The transducer body is, for example made of stainless steel, titanium or aluminium, and encloses a cavity **307** which contains liquid to be aerosolized. The inside of the filling chamber **302** is conical so that liquid flows under gravity into the upstream end **306a** of the transducer body and down into the cavity. Together, the filling chamber **302** and cavity **307** form a reservoir for the liquid.

The membrane **309** is positioned at the downstream end **306b** of the transducer body **306.** The holes in the membrane may be formed by electroforming or by laser drilling, with openings normally in the range from about 1 µm to about 10 µm. Without vibration of the membrane, the balance of pressures, the shape of the holes and the nature of the material used for the membrane are such that the liquid does not seep out through the membrane. However, vibration of the membrane leads to the formation and emission of aerosol droplets through the holes. The membrane may be made of plastic, silicon, ceramic or more preferably metal, and may be affixed onto or into the downstream end of the transducer body by various means, such as gluing, brazing, crimping or laser welding. Optionally, the membrane at least partially forms a dome in its central region, which causes the jet of nascent aerosol droplets to diverge and hence reduces the risk of droplet coalescence.

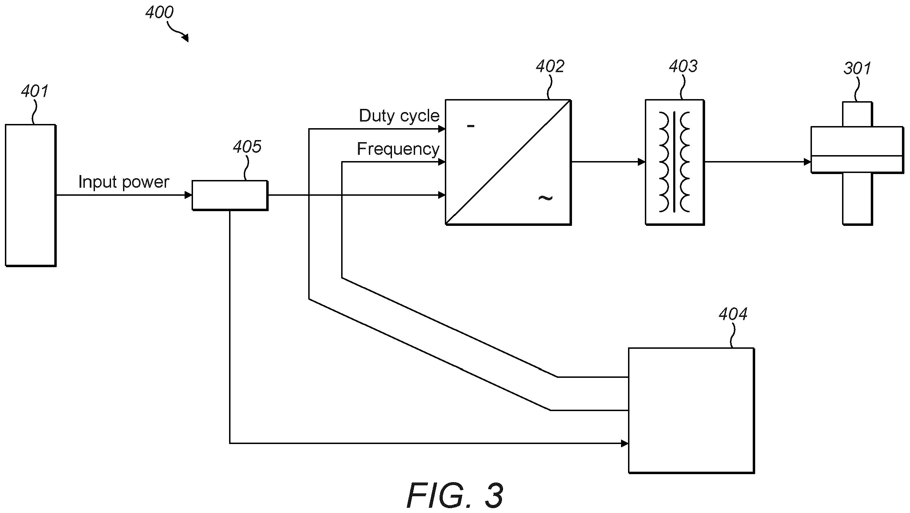

A driver circuit **400,** shown schematically in Figure 3, generates the driver signal that excites the piezoelectric element and hence causes the membrane to vibrate, typically at a frequency in the range of 50 - 200 kHz. The input dc power is provided by a battery **401.** This is converted into an ac driving voltage by a power converter **402** and a transformer **403.** A closed-loop controller **404** controls the power supplied to the aerosol generator **301** by pulse width modulation, by varying the duty cycle, i.e. the fraction of time for which the power is supplied to the aerosol generator. The controller **404** inputs the driving frequency and the duty cycle to the power converter **402.** The controller **404** also measures the current consumed by the aerosol generator **301,** by means of a shunt resistor **405** in series with the input of the power converter **402.** The effective power consumption and the absolute value of the impedance can be derived from the measured current. The aerosol generator is driven using near-resonance driving in which the frequency of the driver signal is as a fixed offset (such as 500Hz or 1 kHz) from the resonant frequency of the aerosol generator (typically around 85kHz).

Excitation of the piezoelectric element causes micronic longitudinal displacements and / or deformations in a direction parallel to the symmetry axis of the transducer body **306.** The transducer body has a region close to the piezoelectric element **308** with a relatively large wall thickness, which serves as a stress concentration zone **306c,** and a region downstream thereof **306d** with a relatively low wall thickness which serves as a deformation amplification zone. This configuration amplifies the vibrations or deformations of the transducer body **306** caused by the piezoelectric element **308.** The piezoelectric element **308** is located at the level of, or adjacent to, the stress concentration zone **306c.** The internal diameter of the transducer body at the deformation amplification zone may be the same as at the stress concentration zone, so that the differences in wall thickness correspond to different external diameters. Alternatively, the external diameter of the transducer body may be constant, while the inner diameters differ at the position of the two zones.

The nebulizer is breath-actuated so that it only generates aerosol when the patient is inhaling. This avoids wasting the aerosol that is generated when the patient is exhaling, as can occur in nebulizers that operate in a continuous manner. A pressure sensor (e.g. a barometric pressure sensor) is located adjacent to, and in pneumatic connection with, the channel in the base unit between the air inlet opening(s) and the air outlet opening **102.** The pressure sensor measures the pressure in the channel, and sends a signal representing the pressure to the controller. When the patient begins to inhale on the mouthpiece, the pressure in the channel drops. If the pressure drops below a certain value, the controller determines that the patient has begun to inhale, and causes the piezoelectric element, and hence the membrane to vibrate, so that aerosol droplets are generated.

When the nebulizer is operated, the aerosol generated by the membrane **309** is released into the channel **205.** Air enters through the air inlets in the base unit and passes through the channel in the base unit, the air outlet opening **102,** and the air inlet opening **201** of the mouthpiece component, and into the channel **205** where mixes it with the aerosol. The air and aerosol then flow along the channel **205,** out through the aerosol outlet opening **203** of the mouthpiece and into the patient's airway.

The controller stops the aerosol generation when a pre-set length of time (for example 3s) has elapsed since the aerosol generation started. The pre-set length of time may correspond to the length of a typical inhalation, and may be configurable by the patient. Alternatively, the pre-set length of time may be shorter than a typical inhalation, so that in the final part of the inhalation, the patient receives air but no aerosol. This ensures that the aerosol reaches the central and lower parts of the patient's airway, but is not delivered to the patient's upper airway (e.g. the throat) where it would be ineffective. However, the controller could alternatively detect when the patient ceases to inhale by sensing the increase in pressure in the channel, and then stop aerosol generation.

The resonant frequency of the aerosol generator changes over the duration of a treatment as the amount of liquid in the reservoir decreases. In order to maintain a fixed offset between the driver signal frequency and the resonant frequency, it is necessary to measure the resonant frequency at intervals throughout operation of the aerosol generator, for example every 0.5s. This is done by scanning the frequency of the driver signal across a range of frequencies from below the resonant frequency to above it, for example from about 10 or 15 kHz below the resonant frequency to about 10 or 15 kHz above the resonant frequency, such as from 75 kHz to about 100 kHz in steps of 0.1kHz. At each frequency, an electrical parameter which relates to the vibration of the aerosol generator is measured, for example the current consumption of the aerosol generator. The resulting graph of current as a function of frequency (the spectrum) has a peak at the resonant frequency of the aerosol generator. The scans take, for example, about 70 ms to perform. During the scans, the aerosol generator does not operate at the optimum frequency, so the aerosol output rate drops. Consequently, almost all of the aerosol is generated in the time between the scans (430 ms in this case).

The point at which the membrane becomes dry can be determined from the scans, for example from changes in the shape of the spectrum as a function of time, or in comparison to a standard spectrum, as described, for example in US2006/0102172, US9272101, WO2014/062175 and WO2015/091356. However, as discussed above, these methods may produce erroneous results as a result of variations in the hardware, changes in the hardware over the lifetime of the nebulizer and changes in external conditions.

The invention is based on a different effect which is independent of these variations, and so is more reliable. When the nebulizer is switched off, the liquid in the cavity has a flat surface with a meniscus around the edge. When the nebulizer is switched on and the aerosol generator is vibrated at or close to its resonant frequency, a standing wave is formed in the liquid within about 50ms. The inverse effect is observed when vibration is stopped, although it takes longer (about 1s) for the wave to dissipate and the liquid surface to become flat again.

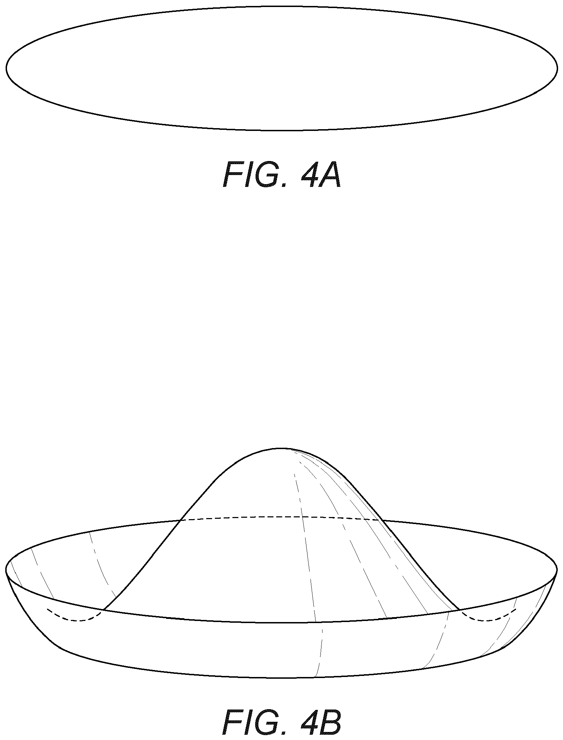

Figure 4 shows schematic drawings of the surface of the liquid in the reservoir of a nebulizer of Figure 1. Figure 4A shows the liquid before vibration was started; the surface is flat, i.e. there is a uniform distribution of the liquid across the transducer body. Figure 4B shows the surface during vibration; the liquid has formed a standing wave with a peak in the centre.

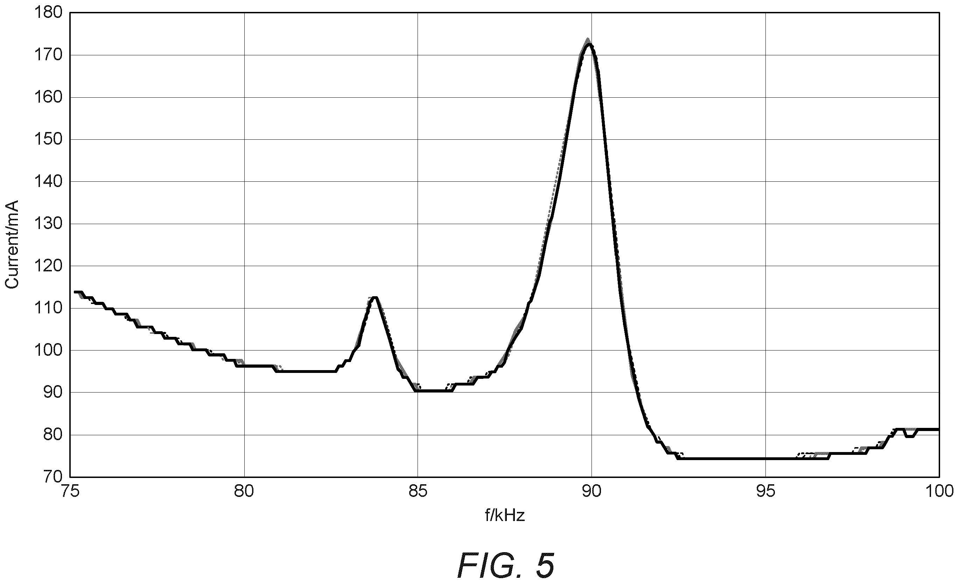

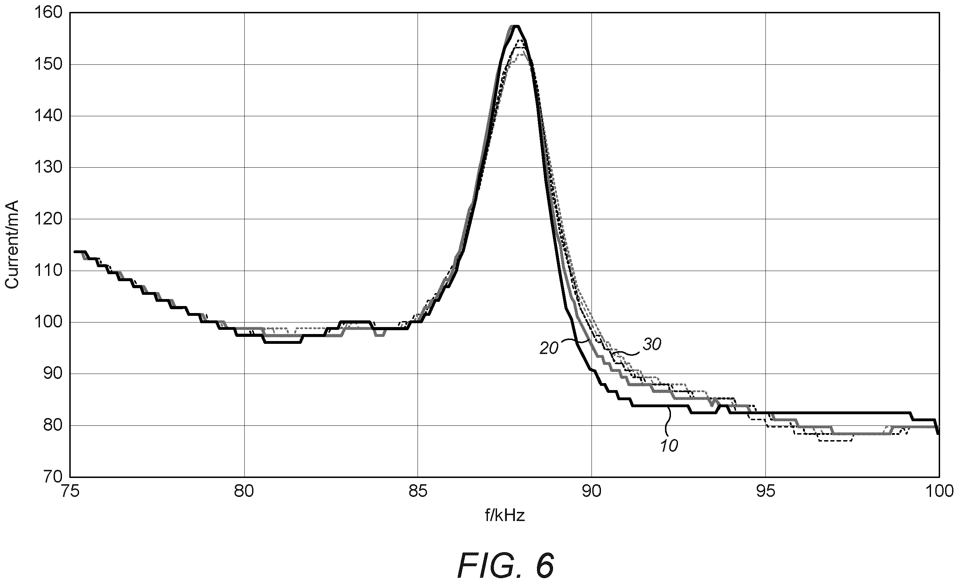

Figures 5 and 6 show spectra obtained with the nebulizer of Figure 1, by vibrating the membrane at a series of different frequencies from 75 kHz to 100 kHz in steps of 0.1khZ with a constant duty cycle, and measuring the current consumption of the power converter at each frequency. Seven scans, each taking 70ms, were performed at 500ms intervals. Between the scans, the membrane was vibrated at its normal near-resonant driving frequency for 430ms.

Figure 5 shows the resulting spectra when no liquid was present. The main peak at 89.5kHz, at which the maximum current consumption occurs, is the resonant frequency of the aerosol generator. There is also a smaller peak at about 83 kHz, which is the resonant frequency of the membrane. No liquid is present so a standing wave is not formed; consequently, the electro-mechanical characteristics of the aerosol generator do not change between the scans. Thus, although each spectrum is plotted as a separate line, they almost exactly overlie each other, and it is not possible to distinguish between them.

Figure 6 shows the spectra obtained when liquid was present. There are differences between the general shape of the spectra compared to those of Figure 5. Firstly, the main resonance peak is at a lower frequency (86.5 kHz) and is slightly broader than in Figure 5, i.e. the resonance is slightly less sharp when liquid is present. These changes in the main resonance peak over the course of a number of inhalations form the basis for some known methods of empty detection. Secondly, the smaller peak has disappeared.

Moreover, in contrast to Figure 5, there are also clearly visible differences between the spectra in Figure 6, due to the re-distribution of liquid as the standing wave is formed when the aerosol generator is switched on. The resonance peak moves to a slightly higher frequency, and becomes slightly broader in each successive spectrum. The largest difference is between the first spectrum **10** and the second spectrum **20.** The third **30** and subsequent spectra are similar to each other, since the liquid had already been re-distributed when these were measured.

Since the these changes only occur when liquid is present, they can be used to distinguish between wet and dry states of the membrane. This forms the basis of the present invention. Thus, instead of comparing a measured value or spectrum with a pre-set value or spectrum, or comparing spectra obtained in different inhalations to identify changes that occur over the course of a treatment, the invention compares a spectrum obtained when aerosol is not being generated with a spectrum obtained after aerosol generation has begun. If no liquid is present, there is no standing wave so the spectra before and during vibration are the same. However, if liquid is present, the spectra are different: the first spectrum reflects the initial, flat liquid surface and the subsequent spectrum reflects the standing wave.

The first spectrum in Figure 6 (at t = 0s, before aerosol generation) can be compared with one (or more) of the subsequent spectra, preferably the second spectrum (at t = 0.5s). The effect is most clearly seen between the first and second spectra because most of the redistribution of liquid occurs quickly, typically within about 50ms, i.e. before the second spectrum is obtained. This difference is also present when comparing the first spectrum with any subsequent spectrum. However, the spectra are also affected by the liquid fill level in the reservoir. There is very little change in the fill level between the times at which the first and second spectra are recorded. In contrast, if the first spectrum is compared with, for example the seventh spectrum (t = 3s), the difference between them would reflect the decrease in the liquid fill level as well as the formation of the standing wave. Thus while the comparison could also be made using subsequent spectra, this is less preferred because these spectra reflect a combination of the two effects.

The degree to which two spectra match each other can be represented by an "overlap function". The overlap function can be calculated as the reciprocal of the sum of the absolute value of the difference between the spectra at each frequency. Thus, when the spectra differ (e.g. the first and second spectra in Figure 6), the sum of the absolute differences is large, and its reciprocal is small; hence the overlap function has a low value. On the other hand, when the spectra are very similar (as in Figure 5) the overlap function has a higher value.

A representative treatment operation was performed using the nebulizer of Figure 1 to nebulize 4mL of 0.9% saline solution. The treatment operation consisted of 195 inhalations, i.e. about 20 µL of solution was nebulized in each inhalation. Aerosol generation was started when the patient's inhalation was detected and continued for 3s in each inhalation. Seven scans were performed (at t=0, 0.5s, 1s, 1.5s, 2s, 2.5s and 3s) during each inhalation. Each scan took 70ms. The resonant frequency was determined from the peak in each scan. Between the scans, the membrane was vibrated for 430ms at a frequency 470 Hz above the resonant frequency, as determined from the immediately preceding scan.

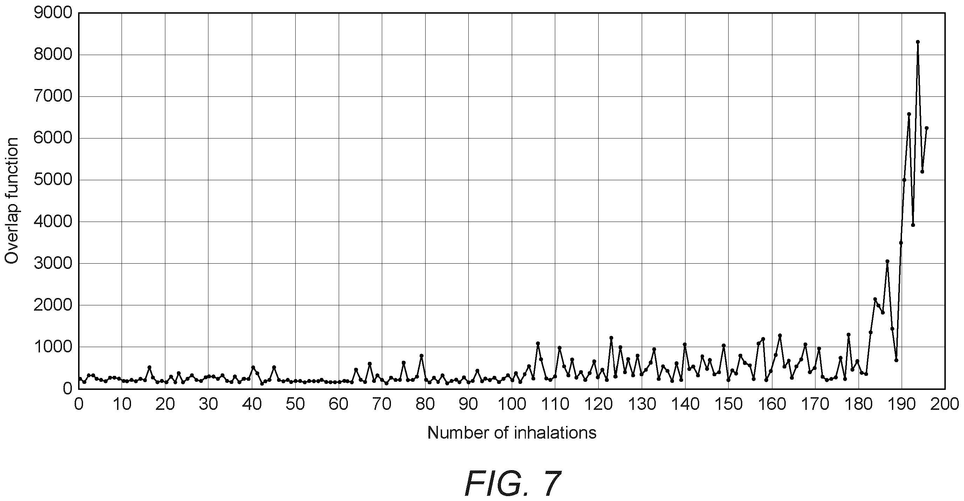

Figure 7 shows a graph of the overlap function for each inhalation in the representative treatment operation. The overlap function is approximately constant at a low value (mostly below 1000) for the first 180 inhalations. This indicates that the first and second spectra differ in these inhalations, because a standing wave is formed in the liquid when the membrane starts to vibrate. Thereafter, the value of the overlap function rises sharply, indicating that the first and second spectra are becoming more similar in breaths 180 - 190, i.e. the standing wave effect is disappearing because there is very little liquid remaining.

A pre-set threshold can be used to determine the point at which the reservoir no longer contains liquid. A threshold value of e.g. 5000 would be suitable in Figure 7. The determination of when the liquid has been used up can be made more robust by only deciding that the reservoir is empty if the value of the overlap function is greater than the threshold for a number of consecutive breaths (e.g. three or five breaths). This prevents incorrect determinations resulting from noise or an erroneous measurement in the overlap function.

Using the first and second spectra means that the overlap function can be calculated slightly sooner than if a subsequent spectrum were used, because the time delay between the first and second spectra (0.5s) is smaller than, for example, between the first and seventh spectra (3s). This has the advantage that the determination of when the threshold has been crossed can be made earlier (by 2.5s in this example), so that the vibration of the membrane is stopped as soon as possible.

The invention is particularly suitable for breath-actuated nebulizers, since the vibrator is necessarily operated intermittently, i.e. only when the patient inhales. It could also be used in nebulizers that normally operate continuously, by introducing periods in which the vibrator is switched off. The duration of the off periods should be at least about 0.5s, preferably 1s, in order to allow the liquid to return to a flat surface before vibration is re-commenced.

The overlap function in Figure 7 was obtained by comparing the spectra immediately before and during a period of aerosol generation, so that the difference between them reflects the formation of a standing wave when the membrane starts to vibrate. In other words, a spectrum obtained during a period of aerosol generation is compared with a spectrum obtained during the preceding period of little or no aerosol generation. However, the overlap function could equally be obtained by comparing a spectrum obtained during a period of aerosol generation with a spectrum obtained during the succeeding period of little or no aerosol generation. The difference between the spectra during and after aerosol generation reflects the dissipation of the standing wave when the membrane ceases to vibrate. This requires that sufficient time has elapsed after vibration ceases before the spectrum is measured. Typically the standing wave takes longer to dissipate (500 - 1000ms) than it takes to form (about 50ms). This is not a concern in a breath-actuated inhaler, since the time between inhalations is usually more than 1s. However, in a nebulizer which is not breath-actuated, the periods in which the membrane is not vibrated would need to be longer to allow for dissipation, which would result in a reduction in the overall aerosol output rate.

The invention is especially suitable for nebulizers of the type shown in Figure 1, in which regular scans are performed in order to determine the resonant frequency. The method of the invention can use the spectra from these scans, and extract additional information from them. Hence implementing the invention requires only some additional analysis of the spectra, and there is no need to change the way in which the nebulizer is operated.

The principle of the invention applies to any vibrating membrane nebulizer in which the membrane is in contact with a liquid reservoir in which a standing wave can be formed. Thus the invention can be used with other types of nebulizer, for example those described in WO2012/046220, WO2015/193432, WO2015/091356, US2006/0102172 and US9027548. These nebulizers do not have a transducer in the form of a hollow tubular body. Instead, the membrane is mounted directly on the piezoelectric element, or there is an annular, planar support member on which the membrane and / or the piezoelectric element are mounted.

The method employed in the device of the invention could be used instead of, or in addition to other empty detection methods (for example as in US2006/0102172, US9272101, WO2014/062175 and WO2015/091356 which measure the changes in an electrical parameter as the volume of liquid decreases over time) to provide a combined decision process for determining whether membrane is dry. Since the method employed in the device of the invention relies on a completely different effect from these other methods, it provides completely independent information on whether liquid is present. Thus the combination of the method employed in the device of the invention and a different method provides particularly robust empty detection.

## Claims

1. A breath-actuated inhalation device comprising an aerosol generator (301) having a vibrator (308) and a membrane (309), a reservoir (302,307) for liquid to be aerosolized which is in fluid communication with the membrane, and a controller (404) that provides a driver signal to drive the vibrator so that the membrane vibrates and generates an aerosol, wherein the controller is configured to:
• drive the vibrator intermittently so that the aerosol generator repeatedly undergoes periods of aerosol generation during a patient's inhalations and periods of little or no aerosol generation preceding and/or succeeding the inhalations;
**characterized in that** the controller is further configured to:
• perform scans in which the membrane is vibrated at a plurality of frequencies, and in which at least one electrical parameter of the vibrator is measured at the plurality of frequencies to provide a spectrum; wherein the scans are performed during the inhalations and during the periods preceding or succeeding the inhalations;
• compare a spectrum obtained during an inhalation with a spectrum obtained during the period preceding or succeeding that inhalation;
• determine whether liquid is present in the reservoir on the basis of the comparison of the spectra; and
• cease to drive the vibrator if the controller determines that no liquid is present.

2. An inhalation device according to claim 1 wherein the controller is configured to perform a first scan before each inhalation to obtain a first spectrum, to subsequently perform a second scan during each inhalation to obtain a second spectrum, and to compare the first and second spectra.

3. An inhalation device according to claim 1 or claim 2, which comprises a channel (205) having an air inlet opening (201) and an aerosol outlet opening (203), and a pressure sensor which is pneumatically connected to the channel, wherein the controller is configured to detect inhalation by a patient at the aerosol outlet opening on the basis of a signal from the pressure sensor, and to initiate a period of aerosol generation in response to the inhalation.

4. An inhalation device according to claim 3, wherein the controller is configured to initiate a period of little or no aerosol generation at a pre-set time after the period of aerosol generation was initiated.

5. An inhalation device according to any of claims 1 to 4, wherein the aerosol generator has a support member (306) comprising a hollow tubular body having a flange at or close to a first end onto which the vibrator (308) is attached, and a second end (306b) into or onto which the membrane (309) is mounted, and wherein the device comprises a filling chamber (302) above the support member, so that the filling chamber (302) and the hollow tubular body (307) together form the reservoir.

6. An inhalation device according to any of claims 1 to 5, wherein the controller is configured to determine the resonant frequency of the aerosol generator from the spectra, and to drive the vibrator at the resonant frequency, or at a frequency related to the resonant frequency, during the periods of aerosol generation other than the scans.

7. An inhalation device according to claim 6, wherein the plurality of frequencies comprises from about 10 or 15 kHz below the resonant frequency to about 10 or 15 kHz above the resonant frequency, for example from 75 kHz to about 100 kHz .

8. An inhalation device according to any of claims 1 to 7, wherein the controller is configured to compare the spectra by calculating an overlap function.

9. An inhalation device according to claim 8, wherein the controller is configured to determine that no liquid is present in the reservoir if the overlap function is above a threshold value

10. An inhalation device according to claim 9, wherein the controller is configured to cease driving the vibrator if the overlap function is above the threshold value for a plurality of consecutive periods of aerosol generation, such as three or five periods.

## Patentansprüche

1. Atmungsbetätigte Inhalationsvorrichtung, umfassend einen Aerosolgenerator (301) mit einem Schwinger (308) und einer Membran (309), ein Reservoir (302, 307) für zu aerosolierende Flüssigkeit, das mit der Membran in Fluidverbindung ist, und eine Steuerung (404), die ein Treibersignal zum Antrieb des Schwingers bereitstellt, so dass die Membran vibriert und ein Aerosol erzeugt, wobei die Steuerung zu Folgendem ausgestaltet ist:
• intermittierendem Antreiben des Schwingers, so dass der Aerosolgenerator wiederholt Zeiträume mit Aerosolerzeugung während der Inhalationen eines Patienten und Zeiträume mit geringer oder keiner Aerosolerzeugung vor und/oder nach den Inhalationen durchläuft,
**dadurch gekennzeichnet, dass** die Steuerung ferner zu Folgendem ausgestaltet ist:
• Durchführen von Prüfungen, bei denen die Membran bei einer Vielzahl von Frequenzen vibriert wird und bei denen mindestens ein elektrischer Parameter des Schwingers zur Bereitstellung eines Spektrums bei der Vielzahl von Frequenzen gemessen wird, wobei die Prüfungen während der Inhalationen und während der Zeiträume vor oder nach den Inhalationen durchgeführt werden,
• Vergleichen eines während einer Inhalation erhaltenen Spektrums mit einem während des Zeitraums vor oder nach dieser Inhalation erhaltenen Spektrum,
• auf der Basis des Vergleichs der Spektren Bestimmen, ob Flüssigkeit in dem Reservoir vorliegt und
• Stoppen des Antreibens des Schwingers, wenn die Steuerung bestimmt, dass keine Flüssigkeit vorliegt.

2. Inhalationsvorrichtung nach Anspruch 1, wobei die Steuerung dazu ausgestaltet ist, vor jeder Inhalation eine erste Prüfung durchzuführen, um ein erstes Spektrum zu erhalten, danach während jeder Inhalation eine zweite Prüfung durchzuführen, um ein zweites Spektrum zu erhalten, und das erste und das zweite Spektrum zu vergleichen.

3. Inhalationsvorrichtung nach Anspruch 1 oder Anspruch 2, die einen Kanal (205) mit einer Lufteinlassöffnung (201) und einer Aerosolauslassöffnung (203) und einen Drucksensor umfasst, der pneumatisch mit dem Kanal verbunden ist, wobei die Steuerung dazu ausgestaltet ist, auf der Grundlage eines Signals von dem Drucksensor eine Inhalation durch einen Patienten an der Aerosolauslassöffnung zu erfassen und als Reaktion auf die Inhalation einen Aerosolerzeugungszeitraum einzuleiten.

4. Inhalationsvorrichtung nach Anspruch 3, wobei die Steuerung dazu ausgestaltet ist, zu einem voreingestellten Zeitpunkt nach der Einleitung des Aerosolerzeugungszeitraums einen Zeitraum mit geringer oder keiner Aerosolerzeugung einzuleiten.

5. Inhalationsvorrichtung nach einem der Ansprüche 1 bis 4, wobei der Aerosolgenerator ein Stützglied (306) hat, das einen hohlen rohrförmigen Körper mit einem Flansch an einem oder in der Nähe eines ersten Endes, an dem der Schwinger (308) angebracht ist, und einem zweiten Ende (306b) umfasst, in oder an dem die Membran (309) montiert ist, und wobei die Vorrichtung eine Füllkammer (302) über dem Stützglied umfasst, so dass die Füllkammer (302) und der hohle rohrförmige Körper (307) zusammen das Reservoir bilden.

6. Inhalationsvorrichtung nach einem der Ansprüche 1 bis 5, wobei die Steuerung dazu ausgestaltet ist, die Resonanzfrequenz des Aerosolgenerators aus den Spektren zu bestimmen und den Schwinger während der Aerosolerzeugungszeiträume, bei denen keine Prüfungen vorgenommen werden, bei der Resonanzfrequenz oder bei einer mit der Resonanzfrequenz in Bezug stehenden Frequenz anzutreiben.

7. Inhalationsvorrichtung nach Anspruch 6, wobei die Vielzahl von Frequenzen von ungefähr 10 oder 15 kHz unter der Resonanzfrequenz bis ungefähr 10 oder 15 kHz über der Resonanzfrequenz umfassen, und beispielsweise von 75 kHz bis ungefähr 100 kHz.

8. Inhalationsvorrichtung nach einem der Ansprüche 1 bis 7, wobei die Steuerung dazu ausgestaltet ist, die Spektren durch Berechnen einer Überlappfunktion zu vergleichen.

9. Inhalationsvorrichtung nach Anspruch 8, wobei die Steuerung dazu ausgestaltet ist zu bestimmen, dass in dem Reservoir keine Flüssigkeit vorliegt, wenn die Überlappfunktion über einem Schwellenwert liegt.

10. Inhalationsvorrichtung nach Anspruch 9, wobei die Steuerung dazu ausgestaltet ist, das Antreiben des Schwingers zu stoppen, wenn die Überlappfunktion während einer Vielzahl von aufeinanderfolgenden Aerosolerzeugungszeiträumen wie drei oder fünf Zeiträumen über dem Schwellenwert liegt.

## Revendications

1. Dispositif d'inhalation actionné par la respiration comprenant un générateur d'aérosol (301) comportant un vibreur (308) et une membrane (309), un réservoir (302, 307) pour un liquide à transformer en aérosol qui est en communication fluidique avec la membrane, et un dispositif de commande (404) qui fournit un signal d'attaque pour attaquer le vibreur de sorte que la membrane vibre et génère un aérosol, dans lequel le dispositif de commande est configuré pour :
• attaquer le vibreur par intermittence de sorte que le générateur d'aérosol subisse de manière répétée des périodes de génération d'aérosol pendant les inhalations d'un patient et des périodes de génération d'aérosol faible ou nulle précédant et/ou suivant les inhalations ; **caractérisé en ce que** le dispositif de commande est en outre configuré pour :
• effectuer des balayages dans lesquels la membrane est mise en vibration à une pluralité de fréquences, et dans lesquels au moins un paramètre électrique du vibreur est mesuré à la pluralité de fréquences pour fournir un spectre ; dans lequel les balayages sont effectués pendant les inhalations et pendant les périodes précédant ou suivant les inhalations ;
• comparer un spectre obtenu pendant une inhalation avec un spectre obtenu pendant la période précédant ou suivant cette inhalation ;
• déterminer si un liquide est présent dans le réservoir sur la base de la comparaison des spectres ; et
• cesser d'attaquer le vibreur si le dispositif de commande détermine qu'aucun liquide n'est présent.

2. Dispositif d'inhalation selon la revendication 1 dans lequel le dispositif de commande est configuré pour effectuer un premier balayage avant chaque inhalation pour obtenir un premier spectre, pour effectuer ensuite un second balayage pendant chaque inhalation pour obtenir un second spectre, et pour comparer les premier et second spectres.

3. Dispositif d'inhalation selon la revendication 1 ou la revendication 2, qui comprend un canal (205) comportant une ouverture d'entrée d'air (201) et une ouverture de sortie d'aérosol (203), et un capteur de pression qui est pneumatiquement relié au canal, dans lequel le dispositif de commande est configuré pour détecter une inhalation par un patient au niveau de l'ouverture de sortie d'aérosol sur la base d'un signal provenant du capteur de pression, et pour commencer une période de génération d'aérosol en réponse à l'inhalation.

4. Dispositif d'inhalation selon la revendication 3, dans lequel le dispositif de commande est configuré pour commencer une période de génération d'aérosol faible ou nulle à un moment prédéfini après le commencement de la période de génération d'aérosol.

5. Dispositif d'inhalation selon l'une quelconque des revendications 1 à 4, dans lequel le générateur d'aérosol comporte un élément de support (306) comprenant un corps tubulaire creux ayant une bride au niveau ou près d'une première extrémité sur laquelle est fixé le vibreur (308), et une seconde extrémité (306b) dans ou sur laquelle est montée la membrane (309), et dans lequel le dispositif comprend une chambre de remplissage (302) au-dessus de l'élément de support, de sorte que la chambre de remplissage (302) et le corps tubulaire creux (307) forment ensemble le réservoir.

6. Dispositif d'inhalation selon l'une quelconque des revendications 1 à 5, dans lequel le dispositif de commande est configuré pour déterminer la fréquence de résonance du générateur d'aérosol à partir des spectres, et pour attaquer le vibreur à la fréquence de résonance, ou à une fréquence relative à la fréquence de résonance, pendant les périodes de génération d'aérosol autres que les balayages.

7. Dispositif d'inhalation selon la revendication 6, dans lequel la pluralité de fréquences comprend d'environ 10 ou 15 kHz en dessous de la fréquence de résonance à environ 10 ou 15 kHz au-dessus de la fréquence de résonance, par exemple de 75 kHz à environ 100 kHz.

8. Dispositif d'inhalation selon l'une quelconque des revendications 1 à 7, dans lequel le dispositif de commande est configuré pour comparer les spectres en calculant une fonction de chevauchement.

9. Dispositif d'inhalation selon la revendication 8, dans lequel le dispositif de commande est configuré pour déterminer qu'aucun liquide n'est présent dans le réservoir si la fonction de chevauchement est supérieure à une valeur seuil.

10. Dispositif d'inhalation selon la revendication 9, dans lequel le dispositif de commande est configuré pour cesser d'attaquer le vibreur si la fonction de chevauchement est supérieure à la valeur seuil pendant une pluralité de périodes consécutives de génération d'aérosol, telles que trois ou cinq périodes.
